# FASCICULE DE BREVET EUROPEEN

(11) **EP 3 402 423 B1**
(45) Date de publication et mention de la délivrance du brevet: **09.04.2025**
(21) Numéro de dépôt: 17711707.4
(22) Date de dépôt: 13.01.2017
(51) Int. Cl.: A61B 17/70, A61B 17/86

(54) **DISPOSITIF D'IMPLANT**
IMPLANTAT
IMPLANT DEVICE

(30) Priorité: 13.01.2016 FR 1650268
(43) Date de publication de la demande: 21.11.2018
(73) Titulaire: Neuro France Implants, 41160 La Ville-Aux-Clercs (FR)
(72) Inventeur: MOREAU, Patrice, 41270 Boursay (FR); WORNER, Karin, 41360 Epuisay (FR); MISSENARD, Gilles, 75016 Paris (FR); TROPIANO, Patrick, 13008 Marseille (FR); COURT, Charles, 78000 Versailles (FR)
(74) Mandataire: Cabinet Bleger-Rhein-Poupon
(86) Numéro de dépôt international: PCT/FR2017/050074
(87) Numéro de publication internationale: WO 2017/121968

(56) Documents cités:
- WO-A1-2010/148299
- DE-A1- 102013 001 933
- DE-U1- 202012 009 023
- US-A1- 2003 074 002
- US-A1- 2013 253 594
- US-A1- 2014 012 334
- US-A1- 2014 121 703
- US-A1- 2015 196 336

## Description

La présente invention a pour objet un dispositif d'implant pour ostéosynthèse, et notamment pour ostéosynthèse vertébrale.

Plus particulièrement, la présente invention a pour objet un implant pour le maintien d'un élément de distraction ou de compression qui consistent généralement en une barre reliée à des implants au travers de moyens support, ou bien en une plaque percée de trous pour le passage des implants.

Un tel implant est par exemple décrit dans le document FR 2880254. Il est du type consistant en une vis comprenant une tige filetée, ainsi qu'une tête munie d'une empreinte pour son manœuvrement, et qui présente une partie sphérique ou partiellement sphérique destinée à coopérer avec une pièce support apte à pivoter sur ladite tête et à y être immobilisée, ladite pièce support comportant des moyens de fixation et de blocage d'un élément de distraction ou de compression.

Il existe de nombreuses variantes de ces dispositifs d'implant, lesquelles diffèrent essentiellement au niveau des moyens de fixation et de blocage d'une barre de distraction ou de compression.

Cependant, tous les dispositifs d'implant présentent les mêmes inconvénients à savoir essentiellement un ancrage qui peut être peu fiable, ainsi qu'une cicatrisation osseuse pas assez rapide.

Certains ont proposé des implants filetés qui présentent, dans le sens longitudinal, des zones distinctes, notamment en étant de pas différents, comme dans les documents US 2013253594 et US 2014012334, avec pour inconvénient que la partie qui comporte un pas plus long accélère la vitesse déplacement de l'implant, en sorte que le pas de la partie qui comporte un pas plus court a tendance à forer un trou plutôt que de réaliser un taraudage, ce qui nuit à l'ancrage.

On connaît également par le document US 2015196336 un implant qui présente deux parties distale et proximale filetées, lesquels sont de pas égaux, avec toutefois comme caractéristique que ledit pas de la partie proximale filetée est un pas à filet double en sorte que l'effet de forage redouté n'est pas écarté.

Un des principaux problèmes rencontrés après l'implantation d'un implant pédiculaire est la création d'une chambre de mobilité de l'implant dans le pédicule vertébral entre la vis et la partie interne du pédicule. Cette mobilité est due à une lyse cellulaire résultant de la pression intense sur la cellule au moment de l'implantation, entre l'âme de la vis et l'os cortical.

La présente invention a pour but de proposer un dispositif d'implant permettant de remédier aux divers inconvénients précités.

Le dispositif d'implant selon l'invention, utilisé pour le maintien d'un élément de distraction ou de compression qui consiste en une barre reliée à des implants au travers de moyens support, ou bien en une plaque percée de trous pour le passage des implants, et du type comprenant une tige filetée, ainsi qu'une tête munie d'une empreinte pour son manœuvrement et qui présente une partie sphérique ou partiellement sphérique destinée à coopérer avec une pièce support apte à pivoter sur ladite tête et à y être immobilisée, ladite pièce support comportant des moyens de fixation et de blocage d'un élément de distraction ou de compression, et il se caractérise essentiellement en ce que ladite partie filetée comporte deux zones, à savoir une zone extrême distale de profil conique et dont le filet à larges spires est configuré pour os spongieux, et une zone proximale de profil conique d'angle de conicité inférieure à celui de celle de ladite partie distale, et dont le filet est fin et configuré pour la corticale de l'os à savoir qu'il comporte des filets tranchants fins et profonds, tandis que l'âme est plate ; et en ce que les deux filetages sont de même pas ou de pas très proches.

La configuration de la partie proximale, à savoir sa conicité, même faible, et son filetage à spire fine et profonde associé à une âme plate, permet d'éviter une lyse cellulaire et donc d'améliorer le contact. La partie interne du pédicule se voit donc moins comprimée ce qui entraîne une meilleure tenue à court moyen et long terme.

Ce filetage a pour but de présenter une accroche osseuse intra pédiculaire maximum sans compression excessive de la cellule osseuse et permet une ostéointégration rapide. Ce filetage présente la particularité de réaliser une progression intra pédiculaire sans contrainte de pression latérale responsable de fracture pédiculaire en fin de vissage. Ce filetage est exclusivement pédiculaire.

Selon une caractéristique additionnelle du dispositif d'implant selon l'invention, il comporte une canule conformée pour permettre l'introduction d'une broche munie d'un capteur de densité.

Selon une autre caractéristique additionnelle du dispositif d'implant selon l'invention, la zone proximale présente une conicité de 1°, tandis que la zone distale présente une conicité de 4°.

Selon une autre caractéristique additionnelle du dispositif d'implant selon l'invention, le filetage de la zone proximale présente un pas inférieur à celui de la zone distale.

Selon une autre caractéristique additionnelle du dispositif d'implant selon l'invention, la différence de valeurs des pas est inférieure à 20 %.

La zone distale comporte un filetage différent à filets ronds et de même profondeur que le filetage de la partie proximale. Il présente en son extrémité une hélice tranchante. Sa conicité permet une introduction progressive sans contrainte. Il est muni de trois fentes parallèles à la pente pour réaliser une fonction auto-taraudage.

L'hélice tranchante permet l'introduction de l'implant dans l'os aisément sans présenter de micro fracture corticale responsable d'amorce de fracture pédiculaire, et elle réalise une fonction d'auto-perforation.

On notera qu'une vis comportant de telles caractéristiques n'est pas réalisable aisément. Le filetage est ainsi réalisé en deux étapes, dans une première on réalise le filetage de la zone distale au moyen d'un tourbillonneur, puis dans une seconde, on réalise le filetage de la zone proximale par tournage à la plaquette, avec, entre ces deux opérations, une étape de repérage du point d'arrêt de la première opération et du point de départ de la seconde opération.

Les avantages et les caractéristiques du dispositif d'implant selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue en perspective d'un dispositif d'implant selon l'invention.
- la figure 2 représente une vue schématique en plan du même dispositif d'implant.
- la figure 3 représente une vue en plan depuis une extrémité du même dispositif d'implant.

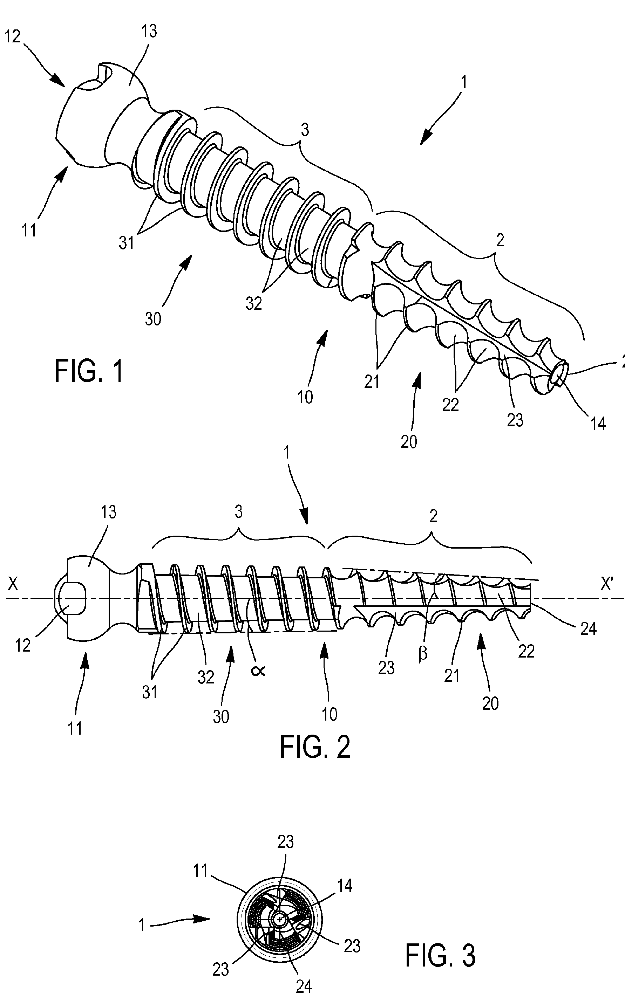

En référence aux figures 1 et 2, on peut voir un dispositif d'implant selon l'invention, comprenant une tige filetée 1 destiné à être associé à une pièce support, non représentée, conçue pour le maintien d'un élément de distraction ou de compression consistant en une barre ou analogue conformément au document FR 2 880 254.

La tige filetée 1 comporte une partie filetée 10, une tête 11 munie d'une empreinte 12 pour son manœuvrement, ainsi qu'une partie 13 partiellement sphérique permettant la coopération en pivotement de la pièce support, non représentée.

Selon l'invention, on peut voir que la partie filetée 10 comporte deux zones, à savoir une zone 2 distale et une zone 3 proximale, s'étendant entre la zone distale 2 et la tête 11.

La zone distale 2, qui s'étend sur les 2/5 de la partie filetée, présente une forme conique, en l'occurrence, préférentiellement mais non limitativement, cette conicité est d'un angle β de 4° par rapport l'axe longitudinal XX'.

La zone proximale 3 est de forme légèrement conique, en l'occurrence, préférentiellement mais non limitativement, elle présente une conicité d'un angle α de 1° par rapport l'axe longitudinal XX'.

Le filetage 30 de la zone proximale 3 présente, sur 3/5 de la longueur totale de l'implant, des spires 31 tranchantes fines et profondes de 0,7 mm, tandis que le fond 32 est plat. Ce filetage 30 a pour but de présenter une accroche osseuse intra pédiculaire maximum sans compression excessive de la cellule osseuse et permet une ostéointégration rapide. Ce filetage 30 présente la particularité de réaliser une progression intra pédiculaire sans contrainte de pression latérale responsable de fracture pédiculaire en fin de vissage. Ce filetage 30 est exclusivement pédiculaire.

La zone distale 2 comporte un filetage 20 différent à spires 21 rondes, de même profondeur que le filetage 30 de la zone proximale 3, et à fond 22 concave.

De manière préférentielle, les filetages 20 et 30 sont de pas identiques. Toutefois, si les filetages 20 et 30 des zones, respectivement distale 2 et proximale 3, sont différents, ils sont alors de valeurs très proches, et le pas du filetage 30 est alors inférieur à celui du filetage 30. En l'occurrence, dans le mode de réalisation représenté, l'écart entre les deux valeurs est de l'ordre de 15%.

La zone distale 2 comporte de plus des entailles 23 d'autotaraudage, au nombre de trois comme cela est visible sur la figure 3.

L'extrémité libre de la zone distale 2 est conformée en une hélice tranchante 24, qui favorise l'introduction dans la matière osseuse.

Sur la figure 3, on peut voir également que l'implant 1 comporte une canule axiale 14, laquelle est destinée à l'introduction d'une broche munie d'un capteur de densité, permettant de déterminer l'emplacement de l'implant.

## Revendications

1. Dispositif d'implant configuré pour maintenir un élément de distraction ou de compression qui consiste en une barre reliée à des implants (1) au travers de moyens support, ou bien en une plaque percée de trous pour le passage des implants (1), et du type comprenant une tige filetée (10), ainsi qu'une tête (11) munie d'une empreinte (12) pour son manœuvrement et qui présente une partie (13) sphérique ou partiellement sphérique destinée à coopérer avec une pièce support apte à pivoter sur ladite tête (11) et à y être immobilisée, ladite pièce support comportant des moyens de fixation et de blocage d'un élément de distraction ou de compression, ladite tige filetée (10) comportant deux zones (2, 3), à savoir une zone extrême distale (2) de profil conique et une zone proximale (3) de profil conique d'angle (α) de conicité inférieure à l'angle (β) de celle de ladite zone extrême distale (2), **caractérisé en ce que** ladite zone extrême distale (2) comporte un filetage (20) configuré pour os spongieux à savoir qu'il présente un filet (21) à spires plus larges que le filetage (30) de la zone proximale (3) tandis que son fond (22) est concave, et ladite zone proximale (3) comporte un filetage (30) configuré pour la corticale de l'os, à savoir qu'il présente une âme (32) plate et des filets (31) plus fins que ceux (21) du filetage (20) de la zone extrême distale (1), formant des spires tranchantes; et **en ce que** les deux filetages (20, 30) sont de même pas.

2. Dispositif d'implant configuré pour maintenir un élément de distraction ou de compression qui consiste en une barre reliée à des implants (1) au travers de moyens support, ou bien en une plaque percée de trous pour le passage des implants (1), et du type comprenant une tige filetée (10), ainsi qu'une tête (11) munie d'une empreinte (12) pour son manœuvrement et qui présente une partie (13) sphérique ou partiellement sphérique destinée à coopérer avec une pièce support apte à pivoter sur ladite tête (11) et à y être immobilisée, ladite pièce support comportant des moyens de fixation et de blocage d'un élément de distraction ou de compression, ladite tige filetée (10) comportant deux zones (2, 3), à savoir une zone extrême distale (2) de profil conique et une zone proximale (3) de profil conique d'angle (α) de conicité inférieure à l'angle (β) de celle de ladite zone extrême distale (2), **caractérisé en ce que** ladite zone extrême distale (2) comporte un filetage (20) configuré pour os spongieux à savoir qu'il présente un filet (21) à spires plus larges que le filetage (30) de la zone proximale (3) tandis que son fond (22) est concave, et ladite zone proximale (3) comporte un filetage (30) configuré pour la corticale de l'os, à savoir qu'il présente une âme (32) plate et des filets (31) plus fins que ceux (21) du filetage (20) de la zone extrême distale (1), formant des spires tranchantes; et **en ce que** les deux filetages (20, 30) sont de pas différents, le filetage (30) de la zone proximale (3) présentant un pas inférieur à celui de la zone distale (2), la différence de valeurs des pas étant inférieure à 20 %.

3. Dispositif d'implant selon la revendication 1 ou la revendication 2, **caractérisé en ce qu'**il comporte une canule (14) conformée pour permettre l'introduction d'une broche munie d'un capteur de densité.

4. Dispositif d'implant selon l'une quelconque des revendications 1 à 3, **caractérisé en ce que** la zone proximale (3) présente une conicité de 1°, tandis que la zone distale (2) présente une conicité de 4°.

5. Dispositif d'implant selon l'une quelconque des revendications 1 à 4, **caractérisé en ce que** le filetage (20) de la zone distale (2) comporte des filets ronds, et de même profondeur que celle du filetage (30) de la partie proximale (3).

6. Dispositif d'implant selon l'une quelconque des revendications 1 à 5, **caractérisé en ce que** la zone distale (2) est munie de trois fentes d'auto-taraudage (23).

7. Dispositif d'implant selon l'une quelconque des revendications 1 à 6, **caractérisé en ce que** son extrémité libre comporte une hélice tranchante (24).

## Patentansprüche

1. Implantatvorrichtung, die dazu konfiguriert ist, ein Distraktions- oder Kompressionselement zu halten, das aus einer Stange, die über Stützmittel mit Implantaten (1) verbunden ist, oder aus einer Platte, die mit Löchern für den Durchgang der Implantate (1) durchbohrt ist, besteht, und die derart ist, dass sie eine Gewindestange (10) sowie einen Kopf (11) umfasst, der mit einer Prägung (12) für seine Handhabung versehen ist und einen kugelförmigen oder teilweise kugelförmigen Teil (13) aufweist, der dazu bestimmt ist, mit einem Stützteil zusammenzuwirken, das auf dem Kopf (11) schwenkbar und dort immobilisierbar ist, wobei das Stützteil Mittel zum Fixieren und Blockieren eines Distraktions- oder Kompressionselements umfasst, wobei die Gewindestange (10) zwei Zonen (2, 3) umfasst, nämlich eine distale äußerste Zone (2) mit konischem Profil und eine proximale Zone (3) mit konischem Profil mit einem Konizitätswinkel (α), der kleiner ist als der Winkel (β) der distalen äußersten Zone (2), **dadurch gekennzeichnet, dass**
die distale äußerste Zone (2) ein für Spongiosa konfiguriertes Gewinde (20) umfasst, d. h., dass es einen Gewindegang (21) mit breiteren Windungen als das Gewinde (30) der proximalen Zone (3) aufweist, während sein Boden (22) konkav ist, und
die proximale Zone (3) ein Gewinde (30) umfasst, das für die Knochenrinde konfiguriert ist, d. h., dass es einen flachen Kern (32) und Gewindegänge (31) aufweist, die schmaler sind als die (21) des Gewindes (20) der distalen äußersten Zone (1), wodurch es schneidende Windungen bildet; und **dadurch, dass** die beiden Gewinde (20, 30) die gleiche Steigung aufweisen.

2. Implantatvorrichtung, die dazu konfiguriert ist, ein Distraktions- oder Kompressionselement zu halten, das aus einer Stange, die über Stützmittel mit Implantaten (1) verbunden ist, oder aus einer Platte, die mit Löchern für den Durchgang der Implantate (1) durchbohrt ist, besteht, und die derart ist, dass sie eine Gewindestange (10) sowie einen Kopf (11) umfasst, der mit einer Prägung (12) für seine Handhabung versehen ist und einen kugelförmigen oder teilweise kugelförmigen Teil (13) aufweist, der dazu bestimmt ist, mit einem Stützteil zusammenzuwirken, das auf dem Kopf (11) schwenkbar und dort immobilisierbar ist, wobei das Stützteil Mittel zum Fixieren und Blockieren eines Distraktions- oder Kompressionselements umfasst, wobei die Gewindestange (10) zwei Zonen (2, 3) umfasst, nämlich eine distale äußerste Zone (2) mit konischem Profil und eine proximale Zone (3) mit konischem Profil mit einem Konizitätswinkel (α), der kleiner ist als der Winkel (β) der distalen äußersten Zone (2), **dadurch gekennzeichnet, dass**
die distale äußerste Zone (2) ein für Spongiosa konfiguriertes Gewinde (20) umfasst, d. h., dass es einen Gewindegang (21) mit breiteren Windungen als das Gewinde (30) der proximalen Zone (3) aufweist, während sein Boden (22) konkav ist, und
die proximale Zone (3) ein Gewinde (30) aufweist, das für die Knochenrinde konfiguriert ist, d. h., dass es einen flachen Kern (32) und Gewindegänge (31) aufweist, die schmaler sind als die (21) des Gewindes (20) der distalen äußersten Zone (1), wodurch es schneidende Windungen bildet; und dadurch, dass die beiden Gewinde (20, 30) unterschiedliche Steigungen aufweisen, wobei das Gewinde (30) der proximalen Zone (3) eine geringere Steigung aufweist als das der distalen Zone (2), wobei der Unterschied der Steigungswerte weniger als 20 % beträgt.

3. Implantatvorrichtung nach Anspruch 1 oder Anspruch 2,
**dadurch gekennzeichnet, dass** es eine Kanüle (14) umfasst, die so geformt ist, dass ein mit einem Dichtesensor ausgestatteter Stift eingeführt werden kann.

4. Implantatvorrichtung nach einem der Ansprüche 1 bis 3,
**dadurch gekennzeichnet, dass** die proximale Zone (3) eine Konizität von 1° aufweist, während die distale Zone (2) eine Konizität von 4° aufweist.

5. Implantatvorrichtung nach einem der Ansprüche 1 bis 4,
**dadurch gekennzeichnet, dass** das Gewinde (20) der distalen Zone (2) runde Gewindegänge und mit der gleichen Tiefe wie das Gewinde (30) der proximalen Zone (3) umfasst.

6. Implantatvorrichtung nach einem der Ansprüche 1 bis 5,
**dadurch gekennzeichnet, dass** die distale Zone (2) mit drei selbstschneidenden Schlitzen (23) versehen ist.

7. Implantatvorrichtung nach einem der Ansprüche 1 bis 6,
**dadurch gekennzeichnet, dass** ihr freies Ende eine schneidende Wendel (24) umfasst.

## Claims

1. Implant device configured to retain a distraction or compression element that consists of a bar which is connected to implants (1) by means of support means, or a plate pierced with holes for implants (1) to pass therethrough, and of the type comprising a threaded shank (10), and a head (11) provided with a recess (12) for maneuvering the device and which has a spherical or partially spherical portion (13) for engaging with a support part which is capable of pivoting on said head (11) and being immobilized thereon, said support part including means for fastening and locking a distraction or compression element, said threaded shank (10) including two zones (2, 3), i.e. a distal end zone (2) with a conical profile and a proximal zone (3) with a conical profile of angle (α) with a taper of less than the angle (β) of that of said distal end zone (2), **characterized in that**
said distal end zone (2) includes a screw thread (20) which is configured for cancellous bone, i.e. it has a thread (21) with wider turns than the screw thread (30) of the proximal zone (3) while its base (22) is concave, and
said proximal zone (3) includes a screw thread (30) which is configured for cortical bone, i.e. it has a flat core (32) and thinner threads (31) than those (21) of the screw thread (20) of the distal end zone (1), forming sharp turns; and **in that** the two screw threads (20, 30) are of the same pitch.

2. Implant device configured to retain a distraction or compression element that consists of a bar which is connected to implants (1) by means of support means, or a plate pierced with holes for implants (1) to pass therethrough, and of the type comprising a threaded shank (10), and a head (11) provided with a recess (12) for maneuvering the device and which has a spherical or partially spherical portion (13) for engaging with a support part which is capable of pivoting on said head (11) and being immobilized thereon, said support part including means for fastening and locking a distraction or compression element, said threaded shank (10) including two zones (2, 3), i.e. a distal end zone (2) with a conical profile and a proximal zone (3) with a conical profile of angle (α) with a taper of less than the angle (β) of that of said distal end zone (2), **characterized in that** said distal end zone (2) includes a screw thread (20) which is configured for cancellous bone, i.e. it has a thread (21) with wider turns than the screw thread (30) of the proximal zone (3) while its base (22) is concave, and
said proximal zone (3) includes a screw thread (30) which is configured for cortical bone, i.e. it has a flat core (32) and thinner threads (31) than those (21) of the screw thread (20) of the distal end zone (1), forming sharp turns; and **in that** the two screw threads (20, 30) have different pitches, the screw thread (30) of the proximal zone (3) having a shorter pitch than that of the distal zone (2), the difference in pitch values being less than 20%.

3. Implant device according to claim 1 or claim 2, **characterized in that** it includes a cannula (14) which is shaped to allow the insertion of a pin provided with a density sensor.

4. Implant device according to any one of claims 1 to 3,
**characterized in that** the proximal zone (3) has a taper of 1°, while the distal zone (2) has a taper of 4°.

5. Implant device according to any one of claims 1 to 4,
**characterized in that** the screw thread (20) of the distal zone (2) includes threads which are round and of the same depth as that of the screw thread (30) of the proximal part (3).

6. Implant device according to any one of claims 1 to 5, **characterized in that** the distal region (2) is provided with three self-tapping slots (23).

7. Implant device according to any one of claims 1 to 6,
**characterized in that** the free end thereof includes a sharp helix (24).
